# EUROPEAN PATENT APPLICATION

(11) **EP 1 389 769 A2**
(43) Date of publication of application: **18.02.2004**
(21) Application number: 03016957.7
(22) Date of filing: 25.07.2003
(51) Int. Cl.: G06F 19/00

(54) **System, method and program for photographing medical image**

(30) Priority: 16.08.2002 JP 2002237619
(71) Applicant: KONICA CORPORATION, Tokyo 163-0512 (JP)
(72) Inventor: Motoki, Wataru, Hachioji-shi, Tokyo 192-8505 (JP)
(74) Representative: Gille Hrabal Struck Neidlein Prop Roos

(57) **Abstract**

A medical image photographing system capable of changing a reader-type of an apparatus for photographing a medical image easily. The system for photographing a medical image by selecting any one reader-type of medical image reading apparatus among a plurality of reader-types of medical image reading apparatuses according to photographing reservation information and controlling the reader-type of medical image reading apparatus, has: a storage for storing photographing conditions and process parameters related to and used under the photographing conditions, an ordering section for ordering the photographing reservation information, a reader-type detecting section for detecting a reader-type capable of photographing a photographic part by searching a photographing condition, a reader-type selecting section for selecting a reader-type to be used for photographing among the detected reader-type, and a photographing control section for controlling the photographing by loading a process parameter corresponding to the photographing condition.

## Description

### BACKGROUND OF THE INVENTION

### Field of the Invention

The invention relates to a system, a method and a program for photographing a medical image with radiation such as X-ray or the like.

### Description of Related Art

Medical image photographing with radiation such as X-ray or the like has been widely used for diagnosing a disease of a patient in a field of medicine. These days, various systems such as a medical image photographing system or the like connected to a network such as a LAN (Local Area Network) or the like built in a hospital, is used for medical purposes.

Generally, the medical image photographing system comprises a reading apparatus for reading an image of a patient with X-ray or the like, and a controller for controlling the reading apparatus. The reading apparatus controlled by the controller has various reader-types such as one for an erect position exclusively, one for a supine position exclusively, one of cassette use or the like. The controller is connected to a reservation apparatus through the above-mentioned network. Reservation information comprising patient information and a photographing condition including a photographic part, a photographing direction, a reader-type of the reading apparatus for photographing or the like, is inputted through the reserving apparatus. The controller controls, based on the reservation information sent from the reservation apparatus, the reading apparatus of the reader-type corresponding to the reservation information.

There are cases that the reservation information is inputted before a radiologist confirms a patient condition, or inputted by an operator who does not know the patient condition. Therefore, when a radiologist confirms the patient condition at a time of photographing, the radiologist often finds it impossible to photograph with the reading apparatus of the reader-type determined based on the reservation information. For example, even though a patient is not able to stand, the reader-type for the erect position exclusively has been reserved. In another case that the ordered reading apparatus such as an erect type is unavailable due to its maintenance or the like, it is necessary to photograph a patient with a cassette and then process the image in an apparatus suitable for the cassette or the like or the like.

In a hospital where a hospital information system (HIS) or a radiology information system (RIS) is built, it is possible for a doctor to input the photographing condition after the doctor confirms the patient condition. However, since the reservation information at the above-mentioned system is converted so as to be processed at the controller, a very complicated photographing condition cannot be set. Therefore, the reader-type of the reading apparatus is specified to be determined only based on the photographic part. As a result, the determined reader-type cannot always be appropriate for the patient condition.

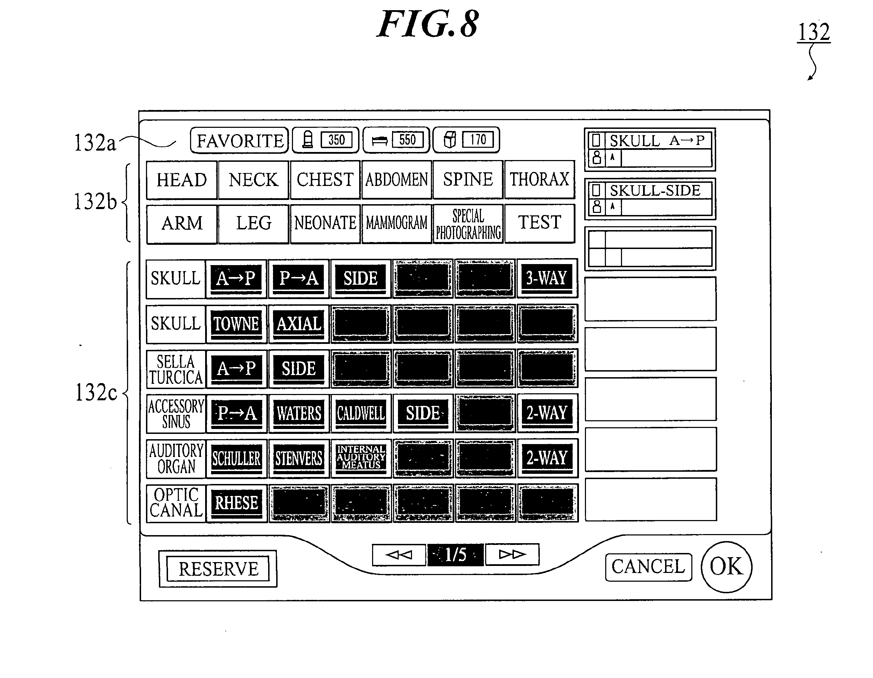

As mentioned above, when the reader-type is not determined corresponding to the patient condition in reservation information, it is necessary to change the reader-type of the reading apparatus at the time of photographing. In an earlier art, in order to change the reader-type of the reading apparatus, it is necessary to go to a condition modifying screen from a photographing order screen at the controller to redetermine all the photographing condition of the reservation information. Therefore, its operation is complicated and time wasting. FIG. 8 shows an example of a condition modifying screen 132 in the earlier art. As shown in FIG. 8, when the reader-type of the reading apparatus is necessary to be changed, it is necessary to reselect the reader-type (selection is made by a button shown in an area 132a), the photographic part (selection is made by a button shown in an area 132b), the photographing direction (selection is made by a button shown in an area 132c) or the like of the photographing condition in the reservation information corresponding to the photographing for which the reader-type is necessary to be changed. As a result, it is complicated.

### SUMMARY OF THE INVENTION

An object of the present invention is to change a reader-type of an apparatus for photographing a medical image easily.

In accordance with a first aspect of the present invention, a medical image photographing system for photographing a medical image by selecting any one reader-type of medical image reading apparatus among a plurality of reader-types of medical image reading apparatuses according to photographing reservation information and controlling the reader-type of medical image reading apparatus, comprises: a storage for storing photographing conditions which are combinations of reader-types of the plurality of medical image reading apparatuses and photographic parts, and process parameters related to the photographing conditions and used under the photographing conditions therein; an ordering section for ordering the photographing reservation information; a reader-type detecting section for detecting at least one reader-type capable of photographing a photographic part by searching a photographing condition including the photographic part according to the photographing reservation information ordered by the ordering section from the storage; a reader-type selecting section for selecting a reader-type which is to be used for photographing among the at least one reader-type detected by the reader-type detecting section; and a photographing control section for controlling the photographing by loading a process parameter corresponding to the photographing condition which is a combination of the reader-type selected by the reader-type selecting section and the photographic part according to the photographing reservation information ordered by the ordering section from the storage.

In accordance with a second aspect of the present invention, a medical image photographing method for photographing a medical image by selecting any one reader-type of medical image reading apparatus among a plurality of reader-types of medical image reading apparatuses according to photographing reservation information and controlling the reader-type of medical image reading apparatus, comprises: storing photographing conditions which are combinations of reader-types of the plurality of medical image reading apparatuses and photographic parts, and process parameters related to the photographing conditions and used under the photographing conditions therein; ordering the photographing reservation information; detecting at least one reader-type capable of photographing a photographic part by searching a photographing condition including the photographic part according to the photographing reservation information ordered among the photographing conditions stored; selecting a reader-type which is to be used for photographing among the at least one reader-type detected; and controlling the photographing by loading a stored process parameter corresponding to the photographing condition which is a combination of the reader-type selected and the photographic part according to the photographing reservation information ordered from the process parameters stored.

In accordance with a third aspect of the present invention, a program, when the program is loaded onto a computer for controlling a medical image photographing system for photographing a medical image by selecting any one reader-type of medical image reading apparatus among a plurality of reader-types of medical image reading apparatuses according to photographing reservation information and controlling the reader-type of medical image reading apparatus, makes the computer execute: storing photographing conditions which are combinations of reader-types of the plurality of medical image reading apparatuses and photographic parts, and process parameters related to the photographing conditions and used under the photographing conditions therein; ordering the photographing reservation information; detecting at least one reader-type capable of photographing a photographic part by searching a photographing condition including the photographic part according to the photographing reservation information ordered among the photographing conditions stored; selecting a reader-type which is to be used for photographing among the at least one reader-type detected; and controlling the photographing by loading a stored process parameter corresponding to the photographing condition which is a combination of the reader-type selected and the photographic part according to the photographing reservation information ordered from the process parameters stored.

According to the system of the first aspect, the method of the second aspect or the program of the third aspect of the present invention, when the photographing conditions which are combinations of reader-types of medical image reading apparatuses and photographic parts, and process parameters used under the photographing conditions are related to each other and stored, and the photographing reservation information is ordered, the photographing condition including the photographic part according to the ordered photographing reservation information is searched from the stored photographing conditions, and the reader-types capable of photographing the photographic part are detected. Then, when the reader-type which is to be used for photographing is selected among the detected reader-types, photographing is controlled by loading the process parameter corresponding to the photographing condition including the selected reader-type and the photographic part according to the ordered photographing reservation information from the stored process parameters. As a result, when the reader-type of the medical image reading apparatus for photographing is necessary to be changed, it is possible to easily change the reader-type with simple operation.

Preferably, the system of the first aspect of the present invention further comprises a code storage for storing the photographic parts and photographic part codes related to the photographic parts, and the reader-types of the plurality of medical image reading apparatuses and reader-type codes related to the reader-types, wherein the storage stores photographing conditions which are combinations of the reader-type codes and the photographic part codes stored in the code storage, and process parameters related to and used under the photographing conditions, and the reader-type detecting section detects at least one reader-type capable of photographing the photographic part by searching a photographing condition including a photographic part code according to the photographing reservation information ordered by the ordering section from the storage.

Preferably, the method of the second aspect of the present invention further comprises storing the photographic parts and photographic part codes related to the photographic parts, and the reader-types of the plurality of medical image reading apparatuses and reader-type codes related to the reader-types, wherein the storing photographing conditions which are combinations of reader-types of the plurality of medical image reading apparatuses and photographic parts, and process parameters related to and used under the photographing conditions therein includes storing photographing conditions which are combinations of the reader-type codes stored and the photographic part codes stored, and process parameters related to and used under the photographing conditions, and the detecting at least one reader-type capable of photographing a photographic part by searching a photographing condition including the photographic part according to the photographing reservation information ordered among the photographing conditions stored includes detecting at least one reader-type capable of photographing the photographic part by searching a photographing condition including a photographic part code according to the photographing reservation information ordered among the photographic part codes stored.

Preferably, the program of the third aspect of the present invention further makes the computer execute storing the photographic parts and photographic part codes related to the photographic parts, and the reader-types of the plurality of medical image reading apparatuses and reader-type codes related to the reader-types, wherein the storing photographing conditions which are combinations of reader-types of the plurality of medical image reading apparatuses and photographic parts, and process parameters related to and used under the photographing conditions therein includes storing photographing conditions which are combinations of the reader-type codes stored and the photographic part codes stored, and process parameters related to and used under the photographing conditions, and the detecting at least one reader-type capable of photographing a photographic part by searching a photographing condition including the photographic part according to the photographing reservation information ordered among the photographing conditions stored includes detecting at least one reader-type capable of photographing the photographic part by searching a photographing condition including a photographic part code according to the photographing reservation information ordered among the photographic part codes stored.

According to the system, the method or the program, the photographic parts and the photographic part codes are related to each other and stored, and the reader-types of the medical image reading apparatuses and the reader-type codes are related to each other and stored, and the photographing conditions which are combinations of the stored reader-type codes and the stored photographic part codes and the process parameters used under the photographing conditions are related to each other and stored. Therefore, by searching the photographing condition including the photographic part code according to the ordered photographing reservation information among the stored photographing conditions, the reader-type capable of photographing the photographic part is detected. Consequently, since the photographic part and the reader-type are coded, it is possible to do the process for changing the reader-type quickly.

Preferably, the system of the first aspect of the present invention further comprises a reader-type displaying section for displaying discriminately the at least one reader-type detected by the reader-type detecting section, wherein the reader-type selecting section selects a reader-type which is to be used for photographing among the at least one reader-type discriminately displayed by the reader-type displaying section.

Preferably, the method of the second aspect of the present invention further comprises displaying discriminately the at least one reader-type detected, wherein the selecting a reader-type which is to be used for photographing among the at least one reader-type detected includes selecting a reader-type which is to be used for photographing among the at least one reader-type displayed.

Preferably, the program of the third aspect of the present invention further makes the computer execute displaying discriminately the at least one reader-type detected, wherein the selecting a reader-type which is to be used for photographing among the at least one reader-type detected includes selecting a reader-type which is to be used for photographing among the at least one reader-type displayed.

According to the system, the method or the program, since the detected reader-type is discriminately displayed, when an operator selects one reader-type, it is possible to clarify a selectable reader-type.

### BRIEF DESCRIPTION OF THE DRAWINGS

The present invention will become more fully understood from the detailed description given hereinafter and the accompanying drawing given by way of illustration only, and thus are not intended as a definition of the limits of the present invention, and wherein:
FIG. 1 is a view conceptually showing a whole structure of a medical image photographing system 100 in the present invention,
FIG. 2 is a block diagram showing a functional structure of a controller 1 in FIG. 1,
FIG. 3 is a table showing an example of data stored in a reader-type code table 171 in FIG. 2,
FIG. 4 is a table showing an example of data stored in a photographic part code table 172 in FIG. 2,
FIG. 5 is a table showing an example of data stored in a photographing condition parameter file 173 in FIG. 2,
FIG. 6 is a flow chart showing a reader-type setting changing process executed by a control unit 11 in FIG. 2,
FIG. 7 is a view of showing an example of a photographing order screen 131 displayed on a display unit 13 in Step S1 in FIG. 6, and
FIG. 8 is a view showing an example of a condition modifying screen 132 in an earlier art.

### EMBODIMENTS OF THE INVENTION

Hereinafter, an embodiment of the present invention will be explained in detail with reference to figures.

Here, at the embodiment explained as follows, a controller 1 has a function as a medical image photographing system claimed in claims of the present invention. Readers 3, 4 and 5 have a function as a medical image reading apparatus claimed in claims of the present invention.

First, a structure of a medical image photographing system 100 will be explained.

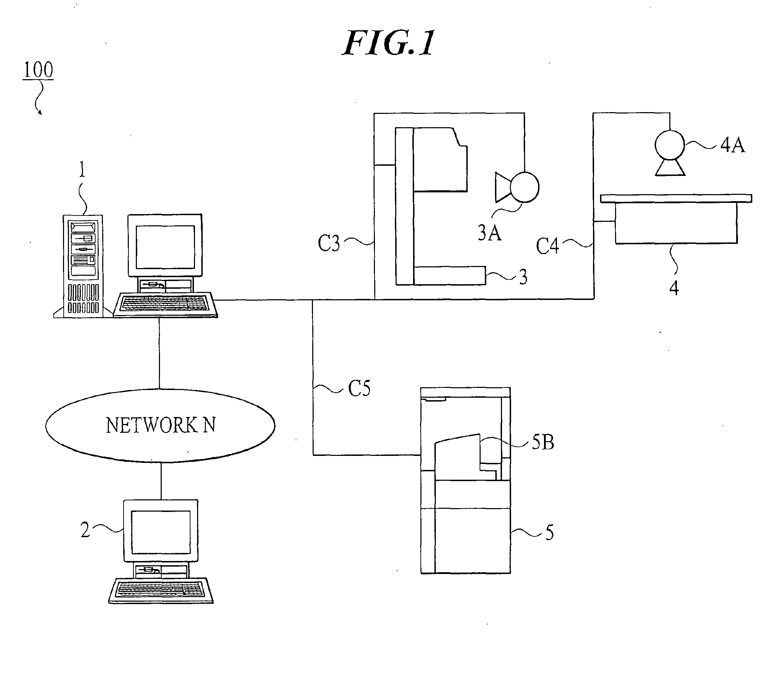

FIG. 1 is a view showing a whole structure of the medical image photographing system 100. The medical image photographing system 100 is a system using a CR (Computed Radiography) or an FPD (Flat Panel Detector) with a semiconductor detector. As shown in FIG. 1, in the medical image photographing system 100, a controller 1 is connected to a reserving apparatus 2 through a network N to be able to send and receive data to each other. Further, the controller 1 is connected to a reader 3 through a cable C3, a reader 4 through a cable C4 and a reader 5 through a cable C5, respectively. The controller 1 controls operation of the readers 3, 4 and 5. Here, FIG. 1 shows an example that one controller 1, one reserving apparatus 2, one reader 3, one reader 4 and one reader 5 are connected to each other but the number of each apparatus can be any.

The controller 1 is connected to the readers 3, 4 and 5 through the cables C3, C4 and C5 respectively, and connected to the reserving apparatus 2 through the network N. When the controller 1 receives reservation information from the reserving apparatus 2, the controller 1 controls the readers 3, 4 and 5 to photographing a medical image according to the reservation information and instructions input by the radiologist. Further, when the controller 1 receives medical image data from the readers 3, 4 and 5, the controller 1 executes an image process, a displaying process or the like.

The reserving apparatus 2 sends the controller 1 the inputted reservation information regarding photographing the medical image through the network N. The reservation information is information for reserving medical image photographing at the medical image photographing system 100. The reservation information is inputted per inspection and includes at least a patient ID and a photographic part of the inspection. Here, the inspection means a group of photographing necessary to be done on a patient at once. The reservation information sent from the reserving apparatus 2 to the controller 1 is converted into photographing reservation information per photographing by referring to a converting table in the controller 1. The photographing reservation information includes a photographing condition such as the photographic part (including a photographing direction), a reader-type or the like.

The reader 3 comprises an X-ray irradiation unit 3A (shown in FIG. 1), and is an image reading apparatus for an erect position exclusively. The reader 3 irradiates X-ray from the X-ray irradiation unit 3A based on an order inputted through the controller 1 connected thereto through the cable C3. Then, radiographic image information made by irradiating X-ray through an object is temporarily accumulated in a sheet-shaped photostimulable phosphor substance plate in which stimulable phosphor substance is laminated on a support member. By scanning the accumulated image with a laser beam, the radiographic image information is sequentially stimulated into luminescence. Then, the luminescence is read by a photoelectric conversion as an image signal. The reader 3 A/D-converts the obtained image signal, and sends the A/D-converted image signal to the controller 1 through the cable C3 as the medical image data. In order to prepare for next photographing, the reader 3 irradiates erasing light on the phosphor substance plate to discharge radiographic energy remaining in the place after the image signal is scanned.

The reader 4 comprises an X-ray irradiation unit 4A (shown in FIG. 1) and is an image reading apparatus for a supine position exclusively. The reader 4, following the same procedure of the reader 3, photographs a medical image based on an order received from the controller 1 through the cable C4. Then, the reader 4 sends the obtained image signal to the controller 1 through the cable C4 as the medical image data.

The reader 5 is an image reading apparatus of cassette use, using a cassette 5B incorporating a photostimulable phosphor substance sheet for accumulating a part of radiographic energy therein. In a photographing room, when X-ray is irradiated toward the cassette 5B from an X-ray irradiation unit unconnected to the reader 5 through an object placed therebetween, radiographic image information of the object made by the X-ray is accumulated in the sheet. When the cassette 5B used for the X-ray irradiation photographing is inserted in the reader 5, the reader 5 irradiates excitation light to the photostimulable phosphor substance sheet in the cassette 5B. Then, stimulated light emitted from the sheet by the excitation light is photoelectrically converted and A/D-converted. Then, the A/D-converted signal is sent to the controller 1 through the cable C5 as the medical image data.

Next, a structure and operation of the controller 1 will be explained in detail with reference to figures hereinafter.

First, the structure will be explained.

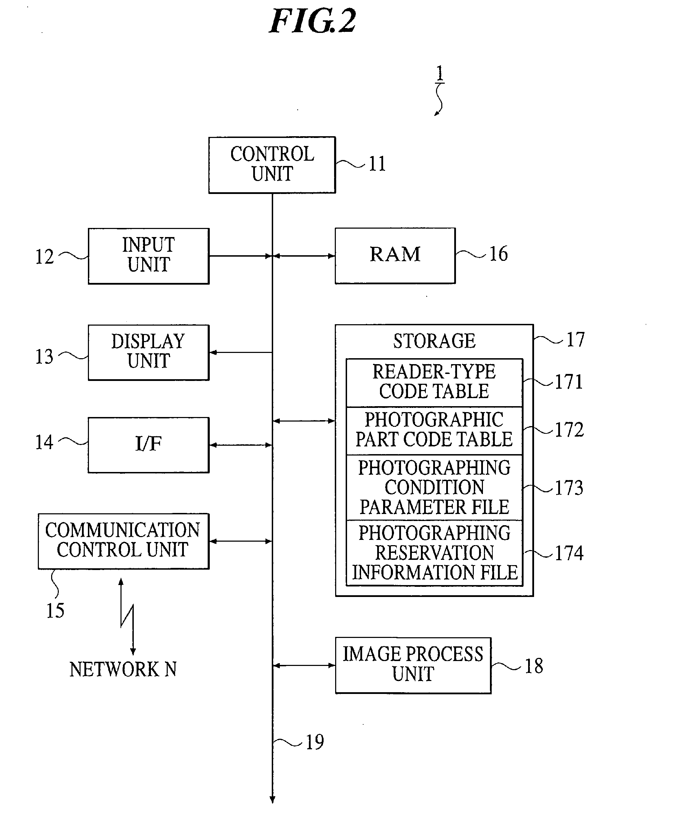

FIG. 2 is a block diagram showing the functional structure of the controller 1. As shown in FIG. 2, the controller 1 comprises a control unit 11, an input unit 12, a display unit 13, an I/F 14, a communication control unit 15, a RAM 16, a storage unit 17 and an image process unit 18. Each unit is connected through a bus 19.

The control unit 11 is composed of a CPU (Central Processing Unit) or the like. The control unit 11 loads a system program or various control programs from the storage unit 17 and develops the programs in the RAM 16 for integrally controlling operations of each unit according to the control program. Further, the control unit 11 executes various processes such as a reader-type setting changing process as follows or the like based on the program developed in the RAM 16. Then, results of the process are temporarily stored in the RAM 16 and displayed on the display unit 13.

For example, when the control unit 11 receives the reservation information per inspection from the reserving apparatus 2, the control unit 11 converts the reservation information by referring to a converting table stored in the storage 17 into the photographing reservation information including the photographing condition per photographing comprising the photographic part, the reader-type or the like. Then, the photographing reservation information is stored in a photographing reservation information file 174. At this conversion, the control unit 11 stores a reader-type code and a photographic part code in the photographing reservation information file 174 as the photographing condition with reference to a reader-type code table 171 and a photographic part code table 172.

Further, in the reader-type setting changing process as follows, when photographing required to change the reader-type of the medical image reading apparatus is ordered on the photographing order screen, the control unit 11 searches a process parameter including the photographic part in the photographing reservation information corresponding to the ordered photographing with reference to a photographing condition parameter file 173. Accordingly, the reader-types capable of photographing the photographic part are detected and reader-type buttons corresponding to the photographing-capable reader-types are displayed discriminately. Then, when one of the discriminately displayed reader-type buttons is pushed to be selected by the input unit 12, the controller 11 changes contents of the photographing reservation information of the photographing in the photographing reservation information file 174 according to the reader-type corresponding to the pushed reader-type button. As a result, the reader-type setting of the medical image reading apparatus for the photographing is changed.

Further, the control unit 11 obtains a reading parameter corresponding to the photographing reservation information of the photographing with reference to the photographing condition parameter file 173. Then, based on the reading parameter, the control unit 11 sends a photographing control signal per photographing to one of the readers 3, 4 or 5 through the corresponding cable C3, C4 or C5 to photograph a medical image. Further, when medical image data made by photographing at the one of the readers 3, 4 or 5 is inputted to the control unit 11 through the cable C3, C4 or C5, the control unit 11 executes an image process based on an image process parameter or an outputting parameter corresponding to the photographing reservation information of the photographing. Then, the controller 11 displays the processed image on the display screen and stores it in the storage unit 17. Here, the control unit 11 has a function as a reader-type detecting section and a photographing control section as claimed in claims of the present invention.

The input unit 12 is composed of a keyboard comprising a cursor key, numeric number input keys, various types of function keys or the like, a mouse as a pointing device or the like. The input unit 12 sends a push signal corresponding to a pushed key on the keyboard and an operation signal of the mouse as an input signal to the control unit 11. Here, in another method, as the input unit 12, it is possible to use one composed of a so-called touch panel wherein input is done by touching a transparent sheet panel covering the display screen of the display unit 13 with a finger or a stylus pen of exclusive use, then the inputted position information is outputted to the control unit 11 as the input signal. Here, the input unit 12 has a function as an ordering section and a reader-type selecting section as claimed in claims of the invention.

The display unit 13 is composed of an LCD (Liquid Crystal Display), a CRT (Cathode Ray Tube) or the like. According to an order of a display signal outputted from the control unit 11, the display unit 13 displays the order inputted through the input unit 12, data or the like thereon. The display 13 has a function as a reader-type displaying section claimed in claims of the present invention.

The I/F 14 is an interface via which the controller 1 is connected to the readers 3, 4 and 5 through the cables C3, C4 and C5.

The communication control unit 15 is composed of a LAN adapter, a router, a TA (Terminal Adapter) or the like. The communication control unit 15 controls communication with each apparatus connected to the network N via a communication line such as a private line, an ISDN (Integrated Services Digital Network) or the like. When the communication control unit 15 receives the reservation information from the reserving apparatus 2 through the network N, the communication control unit 15 outputs the reservation information to the storage unit 17 or the like according to the order of the control unit 11.

The RAM (Random Access Memory) 16 forms a temporary storage area for storing the system program or the control program which is loaded from the storage unit 17 and executable by the controller 1, input or output data, the parameter or the like, for various kinds of processes controlled to be executed by the control unit 11.

The storage unit 17 is composed of a nonvolatile semiconductor memory such as a HDD (Hard Disc Drive) or the like. The storage unit 17 stores the system program corresponding to the controller 1, various kinds of process programs corresponding to the system program, results of the process or the like. Further, the storage unit 17 comprises a memory medium (not shown in FIGS.) wherein a program and data are stored in advance. The memory medium is a nonvolatile memory composed of a magnetic medium, an optical medium or a semiconductor memory. The memory medium is equipped with the storage unit 17 either fixedly or removably. The various kinds of programs are stored in a form of readable program codes. The control unit 11 sequentially executes operation according to the program codes.

In the present invention, the storage unit 17 comprises the converting table (not shown in FIGS.) therein to convert the reservation information per inspection sent from the reserving apparatus 2 into the photographing information per photographing. Further, the storage unit 17 comprises the reader-type code table 171, the photographic part code table 172, the photographing condition parameter file 173 and the photographing reservation information file 174 therein. The storage unit 17 has a function as a storage and a code storage claimed in claims of the present invention.

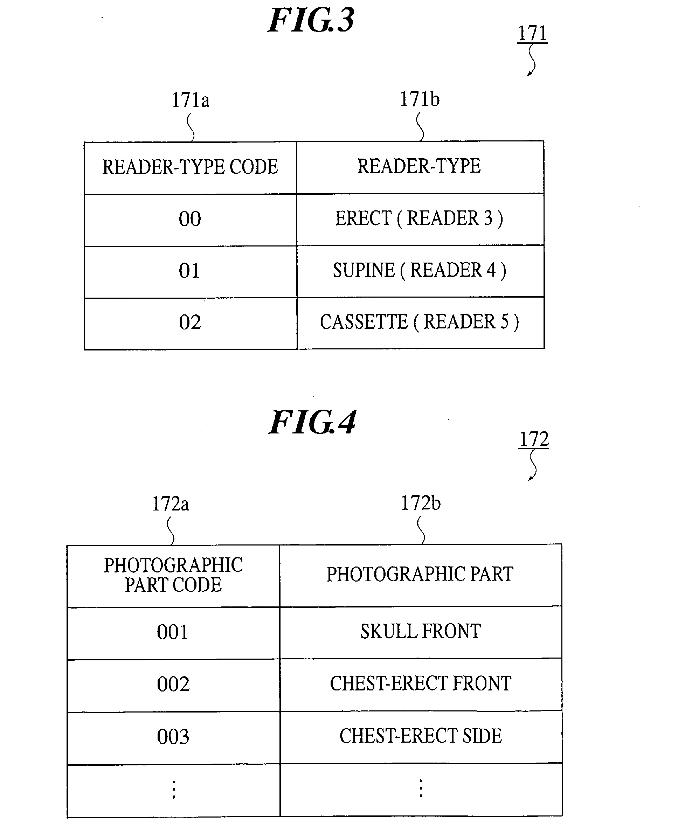

FIG. 3 shows an example of data stored in the reader-type code table 171 for storing the reader-type code and the reader-type of the medical image reading apparatus correspondingly. As shown in FIG. 3, the reader-type code table 171 comprises a reader-type code area 171a for storing an identification code (for example, 00, 01, 02 or the like) uniquely assigned for identifying the reader-type of the reading apparatus as READER-TYPE CODE, and a reader-type area 171b for storing the reader-type (for example, ERECT (READER 3), SUPINE (READER 4), CASSETTE (READER 5)) of the corresponding reading apparatus as READER-TYPE.

FIG. 4 shows an example of data stored in the photographic part code table 172 for storing the photographic part code and the photographic part correspondingly. As shown in FIG. 4, the photographic part code table 172 comprises a photographic part code area 172a for storing an identification code (for example, 001, 002, 003 or the like) uniquely assigned for identifying the photographic part as PHOTOGRAPHIC PART CODE, and a photographic part area 172b for storing the corresponding photographic part (for example, SKULL FRONT, CHEST-ERECT FRONT, CHEST-ERECT SIDE or the like) as PHOTOGRAPHIC PART.

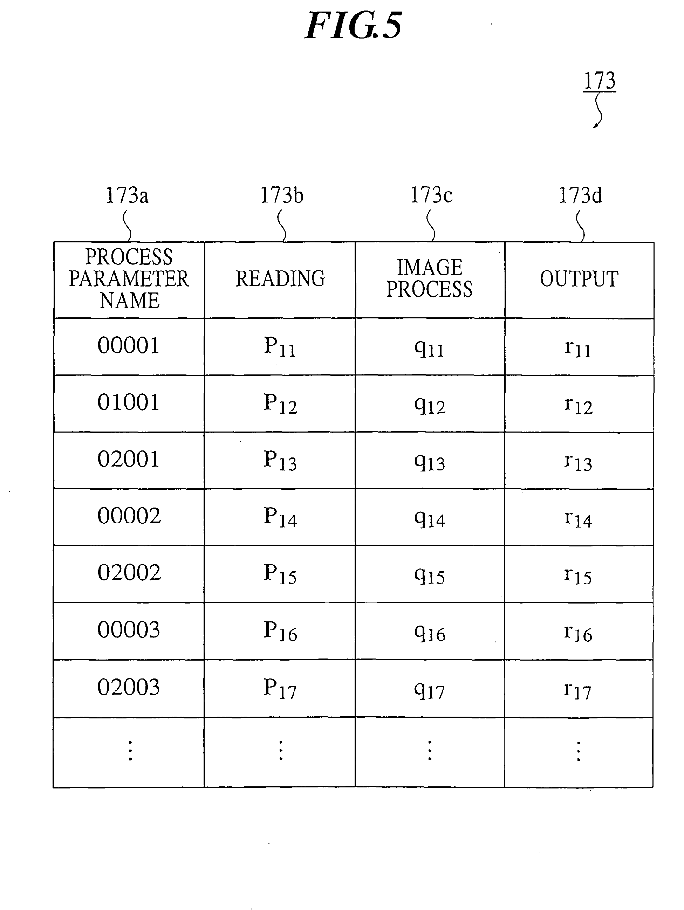

FIG. 5 shows an example of data stored in the photographing condition parameter file 173 for storing a process parameter name which is a combination of READER-TYPE CODE + PHOTOGRAPHIC PART CODE, and process parameters for reading a medical image, processing the image and outputting used upon photographing based on the photographing condition including the combination of READER-TYPE CODE + PHOTOGRAPHIC PART CODE, correspondingly. As shown in FIG. 5, the photographing condition parameter file 173 comprises a process parameter name area 173a for storing data (for example, 00001, 01001, 02001 or the like) showing the combination of READER-TYPE CODE + PHOTOGRAPHIC PART CODE as PROCESS PARAMETER NAME, a reading area 173b for storing a reading parameter (for example, p11, p12, p13 or the like) of the medical image corresponding to the combination of READER-TYPE CODE + PHOTOGRAPHIC PART CODE as READING, an image process area 173c for storing an image process parameter (for example, q11, q12, q13 or the like) corresponding to the combination of READER-TYPE CODE + PHOTOGRAPHIC PART CODE as IMAGE PROCESS, and an output area 173d for storing an output parameter (for example, r11, r12, r13 or the like) corresponding to the combination of READER-TYPE CODE + PHOTOGRAPHIC PART CODE as OUTPUT.

The photographing reservation information file 174 stores patient information and the photographing condition which is a combination of the photographic part and the reader-type per photographing or the like, based on the reservation information sent from the reserving apparatus 2.

The image process unit 18 applies various types of image processes such as a frequency process, a gradation process, a rotation process, an enlarging/reducing process or the like, on medical image data according to the order outputted from the control unit 11. Further, the image process unit 18 executes a compression process for compressing image data with a predetermined encoding method, and an extraction process for decoding and extracting compressed image data.

The network N, which is a communication network built with a private line or an existing general public network, can take a various network form such as a LAN (Local Area Network), a WAN (Wide Area Network) or the like. The network N includes a various communication line network, for example, a phoneline network, an ISDN network, a private line, a mobile communication network, a communication satellite network, a CATV network or the like, and an Internet service provider connected thereto. However, in view of reliability of information management, preferably the network N has security with which only an identified user can access thereto. Further, although each apparatus and each terminal are connected to each other wiredly as shown in FIG. 1, they may be connected wirelessly.

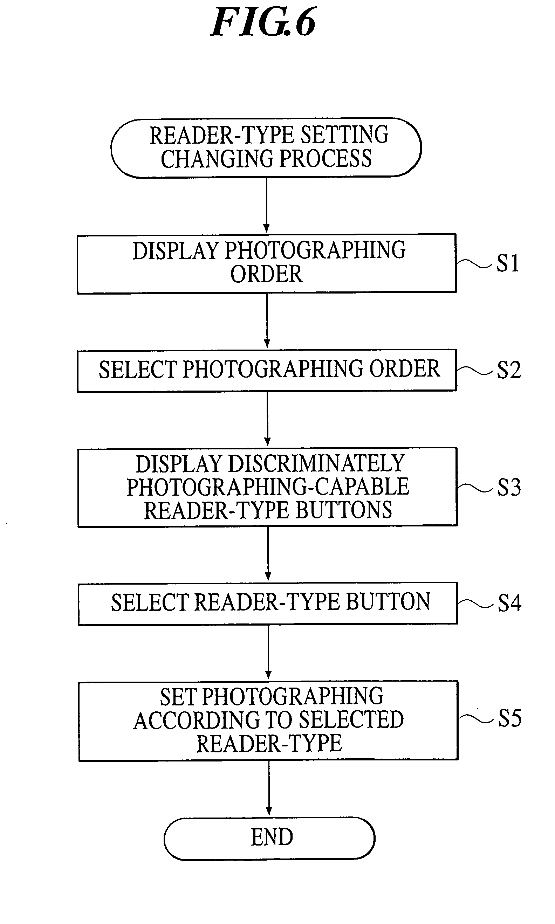

Next, the reader-type setting changing process will be explained with reference to FIG. 6.

FIG. 6 is a flow chart showing a reader-type setting changing process executed by the control unit 11 of the controller 1.

When an order to display the photographing order screen is inputted by operating the input unit 12, the control unit 11 displays the photographing order screen on the display unit 13 (step S1). When one unit of photographing is selected on the photographing order screen, the control unit 11 obtains the reader-type code and the photographic part code in the photographing reservation information of the selected unit of photographing from the photographing reservation information file 174 (step S2). Then, by searching the process parameter name having lower three columns (in other words, the photographic part code) are equal to the photographic part code in the photographing reservation information of the unit of photographing from the photographing condition parameter file 173, the control unit 11 displays the reader-type buttons of the reader-types corresponding the reader-type codes of the searched process parameter name discriminately among reader-type buttons on the photographing order screen (step S3). When one of the displayed reader-type buttons in Step S3 is selected by operating the input unit 12 (step S4), the control unit 11 changes the reader-type in the photographing reservation information of the unit of photographing stored in the photographing reservation information file 174 to the selected reader-type, to set the reader-type for photographing to the selected reader-type.

After the reader-type is changed by the above-mentioned reader-type setting changing process, when the photographing order is inputted through the input unit 12, the control unit 11 loads the process parameter corresponding to the reader-type and the photographic part in the photographing reservation information from the photographing condition parameter file 173 based on the photographing reservation information file 174. Based on the loaded parameter, the control unit 11 reads the medical image. Then, the control unit 11 applies the image process on the read medical image data and outputs the processed data to the output apparatus.

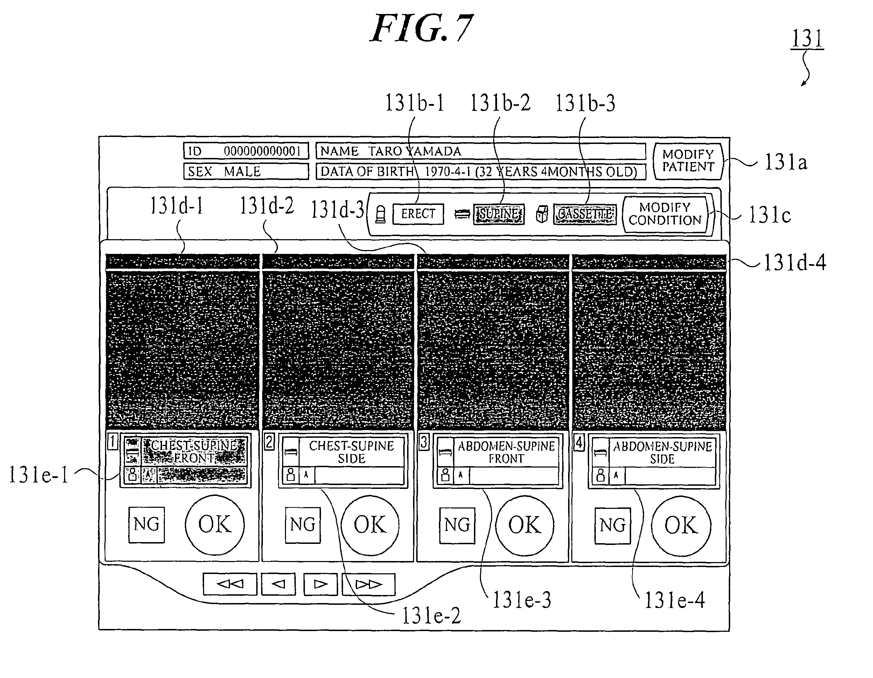

FIG. 7 shows an example of a photographing order screen 131 displayed on the display unit 13 in Step S1 in FIG. 6. As shown in FIG. 7, patient information such as an ID, a name, sex, date of birth or the like of a patient who is to have the photographing is displayed at the top of the photographing order screen 131. A modify patient button 131a located at the right of the patient information is used for going to a screen for modifying the patient information.

Below the patient information, reader-type buttons 131b-1, 131b-2, 131b-3 and a modify condition button 131c are displayed. If the reader-type button 131b-1 is pushed, the reader-type of the medical image reading apparatus for the selected unit of photographing is set ERECT (READER 3). If the reader-type button 131b-2 is pushed, the reader-type of the medical image reading apparatus for the selected unit of photographing is set SUPINE (READER 4). If the reader-type button 131b-3 is pushed, the reader-type of the medical image reading apparatus for the selected unit of photographing is set CASSETTE (READER 5). The modify condition button 131c is used for going to the screen for modifying the photographing condition.

Image displaying areas 131d-1, 131d-2, 131d-3 and 131d-4 are areas for displaying photographed medical images thereon. Below the image displaying areas 131d-1, 131d-2, 131d-3 and 131d-4, photographing reserving buttons 131e-1, 131e-2, 131e-3 and 131e-4 are placed correspondingly. In the photographing reserving buttons 131e-1, 131e-2, 131e-3 and 131e-4, information such as the photographing condition regarding the reserved unit of photographing or the like, is displayed as an icon, and therefore the radiologist can confirm contents of the unit of photographing. The photographing reserving buttons 131e-1, 131e-2, 131e-3 and 131e-4 are buttons for selecting a next unit of photographing. When the unit of photographing is selected by pushing one among the photographing reserving buttons 131e-1, 131e-2, 131e-3 and 131e-4, any of the reader-type buttons 131b-1, 131b-2, 131b-3 corresponding to reader-types capable of photographing the photographic part according to the selected unit of photographing are discriminately displayed. In FIG. 7, since the photographing reserving button of the photographic part displayed as CHEST-SUPINE FRONT is selected, the reader-type buttons 131b-2 and 131b-3 are discriminately displayed because CHEST-SUPINE FRONT can be photographed by SUPINE (READER 4) and CASSETTE (READER 5), corresponding to the discriminately displayed buttons.

As explained above, in the controller 1, when the unit of photographing is selected on the photographing order screen, the control unit 11 searches the process parameter name including the photographic part code in photographing reservation information of the selected unit of photographing with reference to the photographing condition parameter file 173. Then, the controller 11 detects the reader-type capable of photographing the photographic part of the unit of photographing, and discriminately displays reader-type buttons corresponding to the photographing-capable reader-type. When the reader-type button is selected by pushing it based on the reader-type selection, the control unit 11 changes contents of the photographing reservation information of the unit of photographing in the photographing reservation information file 174 according to the pushed reader-type button. Then, the control unit 11 changes the setting of the reader-type of the medical image reading apparatus executing the photographing.

Therefore, since the reader-type capable of photographing the photographic part is detected and displayed on the display screen, when the reader-type is necessary to be changed, it is possible to easily change the reader-type with simple operations.

Here, the description in the above-mentioned embodiment is a preferable example of the present invention, but the present invention is not limited to it.

For example, the contents of the reader-type code table 171, the photographic part code table 172 or the photographing condition parameter file 173 are shown as an example but it is not limited to them. For example, although in the above-mentioned embodiment, the process parameter name of the photographing condition parameter file 173 is the combination of READER-TYPE CODE + PHOTOGRAPHIC PART CODE, it is not limited to the combination but is any one including the combination of READER-TYPE CODE + PHOTOGRAPHIC PART CODE.

In addition, regarding the structure detail and the operational detail of the medical image photographing system 100 and each apparatus composing the medical image photographing system 100, it is possible that various changes may be made without departing the gist of the present invention.

The entire disclosure of Japanese Patent Application No. Tokugan 2002-237619 filed on August 16, 2002 including specification, claims, drawings and summary are incorporated herein by reference in its entirety.

## Claims

1. A medical image photographing system for photographing a medical image by selecting any one reader-type of medical image reading apparatus among a plurality of reader-types of medical image reading apparatuses according to photographing reservation information and controlling the reader-type of medical image reading apparatus, the medical image photographing system comprising:
a storage for storing photographing conditions which are combinations of reader-types of the plurality of medical image reading apparatuses and photographic parts, and process parameters related to the photographing conditions and used under the photographing conditions therein;
an ordering section for ordering the photographing reservation information;
a reader-type detecting section for detecting at least one reader-type capable of photographing a photographic part by searching a photographing condition including the photographic part according to the photographing reservation information ordered by the ordering section from the storage;
a reader-type selecting section for selecting a reader-type which is to be used for photographing among the at least one reader-type detected by the reader-type detecting section; and
a photographing control section for controlling the photographing by loading a process parameter corresponding to the photographing condition which is a combination of the reader-type selected by the reader-type selecting section and the photographic part according to the photographing reservation information ordered by the ordering section from the storage.

2. The system of claim 1, further comprising a code storage for storing the photographic parts and photographic part codes related to the photographic parts, and the reader-types of the plurality of medical image reading apparatuses and reader-type codes related to the reader-types,
wherein the storage stores photographing conditions which are combinations of the reader-type codes and the photographic part codes stored in the code storage, and process parameters related to and used under the photographing conditions, and
the reader-type detecting section detects at least one reader-type capable of photographing the photographic part by searching a photographing condition including a photographic part code according to the photographing reservation information ordered by the ordering section from the storage.

3. The system of claim 1, further comprising a reader-type displaying section for displaying discriminately the at least one reader-type detected by the reader-type detecting section,
wherein the reader-type selecting section selects a reader-type which is to be used for photographing among the at least one reader-type discriminately displayed by the reader-type displaying section.

4. A medical image photographing method for photographing a medical image by selecting any one reader-type of medical image reading apparatus among a plurality of reader-types of medical image reading apparatuses connected thereto according to photographing reservation information and controlling the reader-type of medical image reading apparatus, the method comprising:
storing photographing conditions which are combinations of reader-types of the plurality of medical image reading apparatuses and photographic parts, and process parameters related to the photographing conditions and used under the photographing conditions therein;
ordering the photographing reservation information;
detecting at least one reader-type capable of photographing a photographic part by searching a photographing condition including the photographic part according to the photographing reservation information ordered among the photographing conditions stored;
selecting a reader-type which is to be used for photographing among the at least one reader-type detected; and
controlling the photographing by loading a stored process parameter corresponding to the photographing condition which is a combination of the reader-type selected and the photographic part according to the photographing reservation information ordered from the process parameters stored.

5. The method of claim 4, further comprising storing the photographic parts and photographic part codes related to the photographic parts, and the reader-types of the plurality of medical image reading apparatuses and reader-type codes related to the reader-types,
wherein the storing photographing conditions which are combinations of reader-types of the plurality of medical image reading apparatuses and photographic parts, and process parameters related to and used under the photographing conditions therein includes storing photographing conditions which are combinations of the reader-type codes stored and the photographic part codes stored, and process parameters related to and used under the photographing conditions, and
the detecting at least one reader-type capable of photographing a photographic part by searching a photographing condition including the photographic part according to the photographing reservation information ordered among the photographing conditions stored includes detecting at least one reader-type capable of photographing the photographic part by searching a photographing condition including a photographic part code according to the photographing reservation information ordered among the photographic part codes stored.

6. The method of claim 4, further comprising displaying discriminately the at least one reader-type detected,
wherein the selecting a reader-type which is to be used for photographing among the at least one reader-type detected includes selecting a reader-type which is to be used for photographing among the at least one reader-type displayed.

7. A program, when the program is loaded onto a computer for controlling a medical image photographing system for photographing a medical image by selecting any one reader-type of medical image reading apparatus among a plurality of reader-types of medical image reading apparatuses according to photographing reservation information and controlling the reader-type of medical image reading apparatus, the program making the computer execute:
storing photographing conditions which are combinations of reader-types of the plurality of medical image reading apparatuses and photographic parts, and process parameters related to the photographing conditions and used under the photographing conditions therein;
ordering the photographing reservation information;
detecting at least one reader-type capable of photographing a photographic part by searching a photographing condition including the photographic part according to the photographing reservation information ordered among the photographing conditions stored;
selecting a reader-type which is to be used for photographing among the at least one reader-type detected; and
controlling the photographing by loading a stored process parameter corresponding to the photographing condition which is a combination of the reader-type selected and the photographic part according to the photographing reservation information ordered from the process parameters stored.

8. The program of claim 7, further making the computer execute storing the photographic parts and photographic part codes related to the photographic parts, and the reader-types of the plurality of medical image reading apparatuses and reader-type codes related to the reader-types,
wherein the storing photographing conditions which are combinations of reader-types of the plurality of medical image reading apparatuses and photographic parts, and process parameters related to and used under the photographing conditions therein includes storing photographing conditions which are combinations of the reader-type codes stored and the photographic part codes stored, and process parameters related to and used under the photographing conditions, and
the detecting at least one reader-type capable of photographing a photographic part by searching a photographing condition including the photographic part according to the photographing reservation information ordered among the photographing conditions stored includes detecting at least one reader-type capable of photographing the photographic part by searching a photographing condition including a photographic part code according to the photographing reservation information ordered among the photographic part codes stored.

9. The program of claim 7, further making the computer execute displaying discriminately the at least one reader-type detected,
wherein the selecting a reader-type which is to be used for photographing among the at least one reader-type detected includes selecting a reader-type which is to be used for photographing among the at least one reader-type displayed.
